(19) Europäisches Patentamt
European Patent Office
Office européen des brevets

(11) Veröffentlichungsnummer : **0 154 923**
**B1**

# (12) EUROPÄISCHE PATENTSCHRIFT

(45) Veröffentlichungstag der Patentschrift : **21.12.88**

(51) Int. Cl.⁴ : **C 07 F 7/18, A 23 K 1/16**

(21) Anmeldenummer : **85102449.7**

(22) Anmeldetag : **05.03.85**

## (54) Monosilylierte Aminophenylethylamin-Derivate, Verfahren zu ihrer Herstellung und ihre Verwendung zur Wachstumsförderung.

(30) Priorität : **14.03.84 DE 3409270**

(43) Veröffentlichungstag der Anmeldung : **18.09.85 Patentblatt 85/38**

(45) Bekanntmachung des Hinweises auf die Patenterteilung : **21.12.88 Patentblatt 88/51**

(84) Benannte Vertragsstaaten : **AT BE CH DE FR GB IT LI NL SE**

(56) Entgegenhaltungen :
**EP-A- 0 026 298**
**EP-A- 0 103 830**
**EP-A- 0 104 888**
**EP-A- 0 119 446**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Böshagen, Horst, Dr.**
**Wiesenstrasse 4**
**D-5657 Haan (DE)**
Erfinder : **Stoltefuss, Jürgen, Dl.**
**Parkstrasse 20**
**D-5657 Haan 2 (DE)**
Erfinder : **Berschauer, Friedrich, Dr.**
**Claudiusweg 9**
**D-5600 Wuppertal 1 (DE)**
Erfinder : **de Jong, Anno, Dr.**
**Stockmannsmühle 46**
**D-5600 Wuppertal 1 (DE)**
Erfinder : **Scheer, Martin, Dr.**
**Herberts-Katernberg 7**
**D-5600 Wuppertal 1 (DE)**

**EP 0 154 923 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft monosilylierte Aminophenylethylamin-Derivate, Verfahren zu ihrer Herstellung und ihre Verwendung in der Tierernährung zur Wachstumssteigerung und zur Verbesserung des Fleisch/Fett-Verhältnisses.

Die Verwendung von Futtermittelzusätzen zur Erzielung höherer Gewichtszuwächse und verbesserter Futterausnutzung wird in der Tiernahrung insbesondere bei der Mast von Schweinen, Rindern und Geflügel bereits weitgehend praktiziert. Unsilylierte Aminophenylethylamin-Derivate und ihre Verwendung als Zusatz zur Wachstumsteigerung von Tieren sind aus EP-A-26298 bekannt.

Es wurden die neuen monosilylierten Aminophenylethylamin-Derivate der allgemeinen Formel (I)

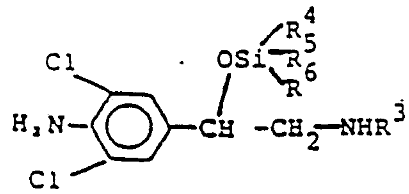

(I)

in der

$R^3$ für einen geradkettigen oder verzweigten ($C_1$-$C_6$) Alkylrest steht, der durch Halogen substituiert sein kann,

$R^4$, $R^5$, $R^6$ für geradkettige oder verzweigte Alkylreste stehen,

und deren physiologisch verträglichen Salze gefunden.

Die Substanzen besitzen ausgezeichnete wachstumsfördernde Wirkungen und bewirken außerdem eine Verbesserung des Fleisch/Fett-Verhältnisses zugunsten von Fleisch.

Bevorzugte Verbindungen sind Phenylethylamin-Derivate der allgemeinen Formel (I), in der $R_3$ einen durch ein oder mehrere Chlor- oder Fluoratome substituierten geradkettigen oder verzweigten $C_1$-$C_6$-Alkylrest bedeutet, sowie deren physiologisch verträglichen Salze.

Man erhält die neuen monosilylierten Aminophenylethylamin-Derivate der Formel (I)

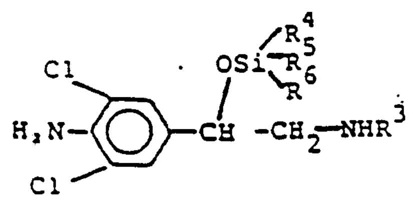

(I)

in welcher

$R^3$ für einen geradkettigen oder verzweigten ($C_1$-$C_6$) Alkylrest steht, der durch Halogen substituiert sein kann,

$R^4$, $R^5$, $R^6$ für geradkettige oder verzweigte Alkylreste stehen,

dadurch, daß man Verbindungen der Formel II

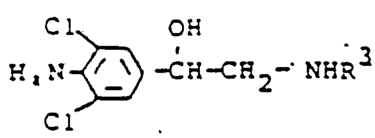

(II)

in der $R^3$ die oben angegebene Bedeutung hat, entweder als Racemat oder in Form der enantiomeren Formen mit geeigneten Silylierungsmitteln der Formel (III)

$$R^4R^5R^6\ Si\text{-}Z \qquad \text{(III)}$$

in der

Z Halogen, CN, $-O-SO_2-CF_3$, $-O-SiR^4R^5R^6$ oder $-O-SO_2-O-SiR^4R^5R^6$ bedeutet,

$R^4$, $R^5$, $R^6$ die oben angegebene Bedeutung besitzen

umsetzt.

Die Ausgangsverbindungen der Formeln (II) und (III) sind entweder bekannt oder können nach

bekannten Methoden hergestellt werden (vgl. z. B. R. E. Lutz and Co-workers, J. Org. Chem. 12, 617-703 (1974)).

Das erfindungsgemäße Verfahren kann in Gegenwart von Verdünnungsmitteln durchgeführt werden. Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethyleter, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z. B. Acetonitril und Propionitril, Benzonitril, Glutarsäuredinitril, darüber hinaus Amide, wie z. B. Dimethylformamid, Dimethylacetamid und N-Methylpyrrolidon, sowie Dimethylsulfoxid, Tetramethylensulfon und Hexamethylphosphorsäuretriamid.

Das erfindungsgemäße Verfahren kann in Gegenwart von Katalysatoren durchgeführt werden. Als Katalysatoren können bevorzugt verwendet werden : Imidazol, Triazol oder Diisopropylethylamin.

Die Reaktionstemperatur wird zwischen etwa 0 °C und 130 °C, vorzugsweise zwischen etwa 20 °C und 100 °C gehalten. Das Verfahren wird vorzugsweise bei Normaldruck durchgeführt.

Die Ausgangsverbindungen der Formeln II und III werden im allgemeinen in etwa äquimolarem Verhältnis eingesetzt. Bevorzugt ist ein Überschuß von 10-200 %, bevorzugt 10-100 %, der Verbindungen der Formel III.

Die Aufarbeitung nach Beendigung der Reaktion erfolgt in an sich bekannter Weise.

Als Tiere, bei denen die Wirkstoffe zur Förderung und Beschleunigung des Wachstums und zur Verbesserung der Futterverwertung eingesetzt werden können, seien beispielsweise folgende Nutz- und Ziertiere genannt :

Warmblüter wie Rinder, Schweine, Pferde, Schafe, Ziegen, Katzen, Hunde, Kaninchen, Pelztiere, z. B. Nerze, und Chinchilla, Geflügel, z. B. Hühner, Gänse, Enten, Truthähne, Tauben, Papageien und Kanarienvögel und Kaltblüter wie Fische, z. B. Karpfen und Reptilien, z. B. Schlangen.

Die Mengen der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,01 bis 50, insbesondere 0,1 bis 10 mg/kg Körpergewicht pro Tag. Die Dauer der Verabreichung kann von wenigen Stunden oder Tagen bis zu mehreren Jahren betragen. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmäßigen oder unregelmäßigen Abständen oral oder parenteral erfolgen.

Die erfindungsgemäßen Wirkstoffe eignen sich besonders für eine parenterale Anwendung, wobei sie mit geeigneten, vorzugsweise nicht wäßrigen, verträglichen Lösungs- bzw. Verdünnungsmitteln in eine anwendbare Formulierung gebracht werden.

Geeignete Formulierungsmittel sind vorzugsweise physiologische Vegetabilien wie z. B. Sesamöl, Erdnußöl oder Maiskeimöl. Diese Öle oder auch andere synthetische Triglyceride wie z. B. Miglycol® oder Myritol® können durch geeignete Zusätze verdickt werden wie z. B. gehärtetes Rizinusöl oder Al-Monostearat. Durch solche Kombinationen kann die Viskosität und damit die Depot-Wirkung in weiten Grenzen variiert werden.

Darüberhinaus sind Implantate aus Silicon bzw. hochmolekularen Polyglycolen oder anderen physiologisch verträglichen Polymeren möglich.

Aus Zweckmäßigkeitsgründen ist häufig eine orale Verabreichung, insbesondere im Rhythmus der Nahrungs- und/oder Getränkeaufnahme derTiere, vorzuziehen.

Die Wirkstoffe können als reine Stoffmischung oder in formulierter Form, also in Mischung mit nicht-toxischen inerten Trägerstoffen beliebiger Art, z. B. mit Trägerstoffen und in Formulierungen, wie sie bei nutritiven Zubereitungen üblich sind, verabreicht werden.

Die Wirkstoffe werden gegebenenfalls in formulierter Form zusammen mit pharmazeutischen Wirkstoffen, Mineralsalzen, Spurenelementen, Vitaminen, Eiweißstoffen, Fetten, Farbstoffen und/oder Geschmacksstoffen in geeigneter Form verabreicht.

Die orale Verabreichung erfolgt vorzugsweise zusammen mit dem Futter und/oder Trinkwasser, wobei je nach Bedarf der Wirkstoff der Gesamtmenge oder nur Teilen des Futters und/oder des Trinkwassers zugegeben wird.

Die Wirkstoffe werden bei oraler Verabreichung nach üblichen Methoden durch einfaches Mischen als reine Stoffmischung, vorzugsweise in fein verteilter Form oder in formulierter Form in Mischung mit eßbaren nicht-toxischen Trägerstoffen, gegebenenfalls in Form eines Praemix oder eines Futterkonzentrates, dem Futter und/oder Trinkwasser beigefügt.

Das Futter und/oder Trinkwasser kann beispielsweise die Wirkstoffe in einer Gewichtskonzentration von etwa 0,01 bis 50, insbesondere 0,1 bis 10 ppm, enthalten. Die optimale Höhe der Konzentration der Wirkstoffe in dem Futter und/oder Trinkwasser ist insbesondere abhängig von der Menge der Futter-und/oder Trinkwasseraufnahme der Tiere und kann durch jeden Fachmann leicht ermittelt werden.

Bei parenteraler Verabreichung hängt die optimale Dosis insbesondere von der Häufigkeit der Verabreichung, der Tierart und dem Alter bzw. Gewicht der Tiere ab.

Die Art des Futters und seine Zusammensetzung ist hierbei ohne Belang. Es können alle gebräuchlichen oder speziellen Futterzusammensetzungen verwendet werden, die vorzugsweise das übliche, für eine ausgewogene Ernährung notwendige Gleichgewicht aus Energie- und Aufbaustoffen einschließlich Vitaminen und Mineralstoffen enthalten. Das Futter kann sich beispielsweise zusammensetzen aus pflanzlichen Stoffen, z. B. Heu, Rüben, Getreide, Getreidenebenprodukten, tierischen Stoffen, z. B. Fleisch, Fetten, Knochenmehl, Fischprodukten, Vitaminen, z. B. Vitamin A, D-Komplex und B-Komplex, Proteinen, Aminosäuren, z. B. DL-Methionin und anorganischen Stoffen, z. B. Kalk und Kochsalz.

Futterkonzentrate enthalten die Wirkstoffe neben eßbaren Stoffen, z. B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentraten können die Wirkstoffe gegebenenfalls auch durch ihre Oberfläche bedeckende geeignete Mittel, z. B. mit nicht-toxischen Wachsen oder Gelatine vor Luft, Licht und/oder Feuchtigkeit geschützt werden.

Beispiel für die Zusammensetzung eines Kükenaufzuchtfutters, das einen erfindungsgemäßen Wirkstoff enthält :

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Praemix ergeben nach sorgfältigem Mischen 1 kg Futter.

In einem kg Futtermischung sind enthalten : 600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $MnSO_4 \times H_2O$, 140 mg $ZnSO_4 \times 7H_2O$, 100 mg $FeSO_4 \times 7H_2O$ und 20 mg $CuSO_4 \times 5H_2O$.

Der Wirkstoff-Praemix enthält die Wirkstoffe in der gewünschten Menge, z. B. 10 mg und zusätzlich 1 g DL-Methionin sowie so viel Sojabohnenmehl, daß 2,5 g Praemix entstehen.

Beispiel für die Zusammensetzung eines Schweineaufzuchtfutters, das einen erfindungsgemäßen Wirkstoff enthält :

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine (Zusammensetzung z. B. wie beim Kükenfutter), 30 g Leinkuchenmehl, 30 g Maiskleberfutter, 10 g Sojaöl, 10 g Zuckerrohrmelasse und 2 g Wirkstoff-Praemix (Zusammensetzung z. B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auf zur Aufzucht und Mast anderer Tiere verwendet werden.

Mit den erfindungsgemäßen Wirkstoffen wurden mehrere Fütterungs- und Stoffwechseluntersuchungen durchgeführt.

Dabei wurden folgende Ergebnisse erhalten :

## Beispiel 1a

a) Tier-Charakteristik und Futter

| | |
|---|---|
| a 1) Ratten, weiblich | |
| a 2) Anzahl | 15 |
| a 3) Zucht | SPF Wistar, Züchtung Hagemann |
| a 4) Gewicht | 150-200 g |
| a 5) Zustand | gut |
| a 6) Futter | |

Rohnährstoffe*

| | |
|---|---|
| Rohprotein | 19,0 |
| Rohfett | 4,0 |
| Rohfaser | 6,0 |
| Asche | 7,0 |
| Wasser | 13,5 |
| N-freie Extraktst. | 50,5 |
| Umsetzbare Energie : | |
| Kcal/kg | 3 100 |
| KJ/kg | 13 000 |

Mineralstoffe*

| | |
|---|---|
| Calcium | 0,9 |
| Phosphor | 0,7 |
| Magnesium | 0,2 |
| Natrium | 0,2 |

Vitamine**

Standard-Diät

| | |
|---|---|
| Vitamin A | 15 000 IE |
| Vitamin $D_3$ | 600 IE |
| Vitamin E | 75 mg |
| Vitamin $K_3$ | 3 mg |
| Vitamin $B_1$ | 18 mg |
| Vitamin $B_2$ | 12 mg |
| Vitamin $B_6$ | 9 mg |
| Vitamin $B_{12}$ | 24 mcg |
| Nikotinsäure | 36 mg |
| Pantothensäure | 21 mg |
| Folsäure | 2 mg |
| Biotin | 60 mg |
| Cholin | 600 mg |
| Vitamin C | 36 mg |

Aminosäuren*

| | |
|---|---|
| Lysin | 0,9 |
| Methionin + Cystin | 0,6 |
| Phenylalanin + Tyrosin | 1,4 |
| Arginin | 1,1 |
| Histidin | 0,4 |
| Tryptophan | 0,2 |
| Threonin | 0,6 |
| Isoleucin | 0,9 |
| Leucin | 1,3 |
| Valin | 0,9 |

Spurenelemente**

| | |
|---|---|
| Magnan | 75,0 |
| Eisen | 135,0 |
| Kupfer | 13,0 |
| Zink | 70,0 |
| Jod | 0,9 |
| Fluor | 9,0 |

* % in der Diät (Mittelwert)
** mg in 1 kg Diät (Mittelwert)

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 17-tägige Hauptperiode durchgeführt, in denen Futteraufnahme, Zuwachs und Futterverwertung bestimmt wurden.

Es wurden folgende Behandlungen geprüft :

b 1) Negativkontrolle (n = 10)

b 2) 25 ppm (Wirkstoff Beispiel 1) (n = 5)

c) Ergebnis (Futteraufnahme, Wachstum, Futterverwertung) während der Hauptperiode (17 Tage)

| | Futter-aufnahme (g) | Zuwachs (g) | Futter-verwer-tung (g/g) |
|---|---|---|---|
| c 1) Negativkontrolle | 226 | 39,1 | 6,80 |
| c 2) 25 ppm (Wirkstoff Bsp. 1) | 320 | 58,6 | 5,46 |

Beispiel 1b

a) Tier-Charakteristik und Futter

| | |
|---|---|
| a 1) Ratten, weiblich | |
| a 2) Anzahl | 15 |
| a 3) Zucht | SPF Wistar, Züchtung Hagemann |
| a 4) Gewicht | 200-235 g |
| a 5) Zustand | gut |
| a 6) Futter | wie in Beispiel 1A |

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 17-tägige Hauptperiode durchgeführt, in denen Futteraufnahme, Zuwachs und Futterverwertung bestimmt wurden.

Es wurden folgende Behandlungen geprüft :

b 1) Negativkontrolle (n = 10)

b 2) 0,2 ppm (Wirkstoff Beispiel 1) (n = 5)

c) Ergebnis (Futteraufnahme, Wachstum, Futterverwertung) während der Hauptperiode (17 Tage)

| | Futter-aufnahme (g) | Zuwachs (g) | Futter-verwer-tung (g/g) |
|---|---|---|---|
| c 1) Negativkontrolle | 291 | 23,9 | 12,17 |
| c 2) 0,25 ppm (Wirkstoff Bsp. 1) | 350 | 42,7 | 8,20 |

Herstellungsbeispiele

Beispiel 1

N-[2-(4-Amino-3,5-dichlor-phenyl)-2-(1,2-dimethyl-propyl-dimethyl-silyloxy)-ethyl]-N-t-butylamin

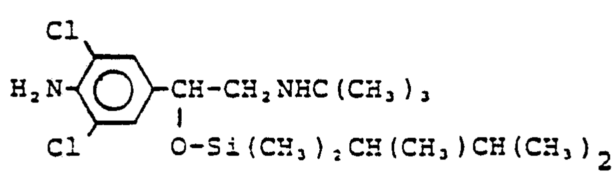

2,77 g (10 mMol) 1-(4-Amino-3,5-dichlor-phenyl)-2-(t-butylamino)-ethanol in 50 ml abs. DMF werden mit 2,04 g (30 mMol) Imidazol versetzt und unter Rühren 3,64 g (22 mMol) Dimethyl-isoamyl-silylchlorid zugetropft. Nach dem Abklingen der leicht exothermen Reaktion wird noch 1 Stunde bei R. T. nachgerührt, dann das DMF im Vakuum abgezogen, der sirupöse Rückstand mit 50 ml Wasser versetzt und 3 × mit je 50 ml Toluol extrahiert. Der Toluol-Extrakt wird noch 3 × mit Wasser ausgewaschen, mit Magnesiumsulfat getrocknet und im Vakuum eingeengt. Der zurückbleibende hellgelbe Sirup wird anschließend 2 Stunden im Hochvakuum (0,01 Torr) evakuiert. 4,02 g (99 % der Theorie) Hellgelbes öl. Das Produkt ist gaschromatographisch rein.

$R_f$-Wert : 0,76.

(DC-Alufolie [Merck], Kieselgel 60 $F_{254}$ ; Laufmittel : Toluol/Ethanol-1/1).

IR ($CHCl_3$) $cm^{-1}$ : 3495 ; 3403 ; 2961 (st) ; 2871 (m) ; 1620 (m) ; 1582 ; 1485 (st) ; 1414 ; 1399 ; 1366 (m) ; 1291 ; 1254 (st) ; 1226 ; 1091 (st) ; 975 (m) ; 925 ; 904 ; 874 ; 837 (st).

Die folgenden Verbindungen wurden wie vorgehend beschrieben hergestellt :

## Beispiel 2

N-[2-(4-Amino-3,5-dichlor-phenyl)-2-(1,2-dimethyl-propyl-dimethylsilyloxy)-ethyl]-N-isopropylamin

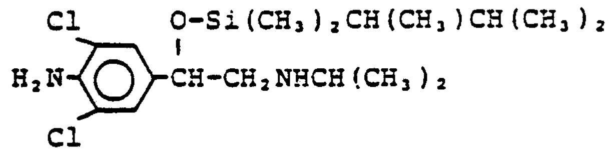

Ausbeute : 70 % der Theorie. Hellgelbes öl. Gaschromatographisch einheitlich.

$R_f$-Wert : 0,68

(DC-Alufolie [Merck], Kieselgel 60 $F_{254}$ ; Laufmittel : Toluol/Ethanol-1/1).

IR ($CHCl_3$) $cm^{-1}$ : 3501 ; 3401 ; 2963 (st) ; 2871 (st) ; 1620 (m) ; 1582 (m) ; 1487 (st) ; 1415 ; 1385 ; 1346 ; 1292 ; 1254 (st) ; 1177 ; 1081 (st) ; 971 ; 920 ; 875 (m) ; 837 (st).

## Beispiel 3

N-[2-(4-Amino-3,5-dichlor-phenyl)-2-(1,2-dimethyl-propyl-dimethylsilyloxy)-ethyl]-N-(2,2-dimethyl)-propylamin

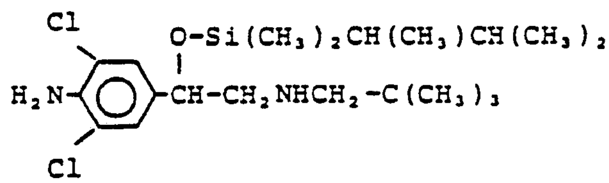

Ausbeute : 78 % der Theorie Hellgelbes öl. Gaschromatographisch einheitlich.

$R_f$-Wert : 0,72

(DC-Alufolie [Merck], Kieselgel 60 $F_{254}$ ; Laufmittel : Toluol/Ethanol-3/1).

IR ($CHCl_3$) $cm^{-1}$ : 3498 ; 3403 ; 2960 (s) ; 2871 ; 2829 ; 2355 ; 1619 (m) ; 1585 (m) ; 1563 ; 1486 (s) ; 1413 ; 1367 (m) ; 1291 ; 1254 (s) ; 1086 (s) ; 1010 ; 974 ; 928 ; 910 (m) ; 872 ; 837 (s) ; 728.

## Beispiel 4

N-[2-(4-Amino-3,5-dichlor-phenyl)-2-(1,2-dimethyl-propyl-dimethylsilyloxy)-ethyl]-N-(1,1-dimethyl)-propylamin

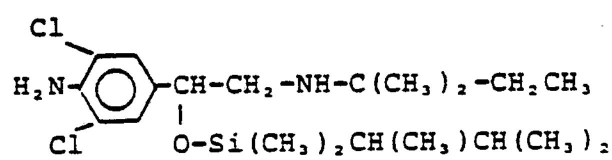

Ausbeute : 86 % der Theorie Hellgelbes öl. Gaschromatographisch einheitlich.

$R_f$-Wert : 0,81

(DC-Alufolie [Merck], Kieselgel 60 $F_{254}$ ; Laufmittel : Toluol/Ethanol-1/1).

IR ($CHCl_3$) $cm^{-1}$ : 3500 ; 3402 ; 2962 (st) ; 1617 (m) ; 1583 (m) ; 1486 (st) ; 1413 ; 1365 ; 1290 ; 1253 (st) ; 1093 (st) ; 980 (m) ; 923 ; 874 (m) ; 836 (st).

Beispiel 5

N-[2-(4-Amino-3,5-dichlor-phenyl)-2-(t-butyl-dimethylsilyloxy)-ethyl]-N-t-butylamin

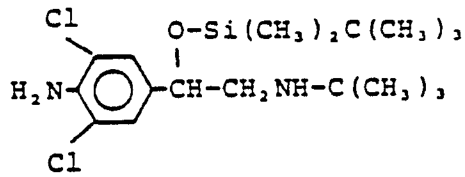

4,16 g (15 mMol) 1-(4-Amino-3,5-dichlor-phenyl)-2-(butylamino)-ethanol werden in 50 ml abs. DMF gelöst, 3,06 g (45 mMol) Imidazol zugegeben und unter Rühren 4,97 g (33 mMol) t-Butyl-dimethylsilylchlorid zugetropft. Die Mischung wurde 1 Stunde bei R.T., 1 Stunde bei 60 °C und 3 Stunden bei 80 °C gerührt. Dann wurde das Lösungsmittel im Vakuum abgezogen, der Rückstand mit 50 ml Wasser versetzt und 3 x mit 50 ml Toluol extrahiert. Der Toluol-Extrakt wurde noch 3 x mit Wasser gewaschen, getrocknet und im Vakuum eingedampft. Der zurückbleibende Sirup wird über 100 g Kieselgel 60 chromatographiert (Laufmittel : 1) Toluol, 2) Tol/EtOH-30/1). Man erhält 5,3 g (90 % der Theorie) einer chromatographisch einheitlichen Fraktion in Form eines gelben Öles.

$R_f$-Wert : 0,65

(DC-Alufolie [Merck], Kieselgel 60 $F_{254}$ ; Laufmittel : Toluol/Ethanol-3/1).

**Patentansprüche**

1. Monosilylierte Aminophenylethylamin-Derivate der allgemeinen Formel (I)

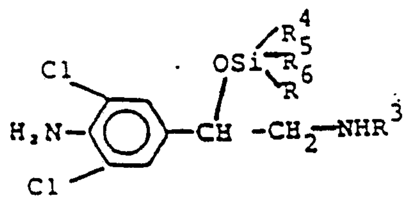

(I)

in der

$R^3$ für einen geradkettigen oder verzweigten ($C_1$-$C_6$) Alkylrest steht, der durch Halogen substituiert sein kann,

$R^4$, $R^5$, $R^6$ für geradkettige oder verzweigte Alkylreste stehen,

und deren physiologisch verträgliche Salze.

2. Verfahren zur Herstellung der monosilylierten Aminophenylethylamin-Derivaten der Formel (I)

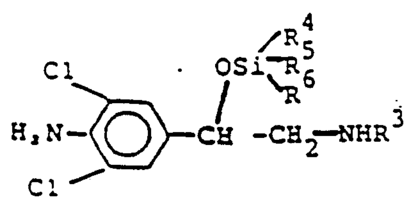

(I)

in welcher

$R^3$ für einen geradkettigen oder verzweigten ($C_1$-$C_6$) Alkylrest steht, der durch Halogen substituiert sein kann,

$R^4$, $R^5$, $R^6$ für geradkettige oder verzweigte Alkylreste stehen,

dadurch gekennzeichnet, daß man Verbindungen der Formel II

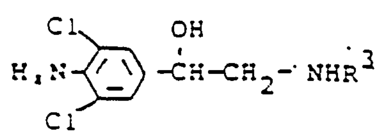

(II)

in der $R^3$ die oben angegebene Bedeutung hat, entweder als Racemat oder in Form der enantiomeren Formen mit geeigneten Silylierungsmitteln der Formel (III)

$$R^4R^5R^6\ Si\text{-}Z \qquad (III)$$

in der

Z Halogen, CN, $-O-SO_2-CF_3$, $-O-SiR^4R^5R^6$ oder $-O-SO_2-O-SiR^4R^5R^6$ bedeutet.

$R^4$, $R^5$, $R^6$ die oben angegebene Bedeutung besitzen

umsetzt.

3. Verwendung von monosilylierten Aminophenylethylamin-Derivaten der Formel I gemäß Anspruch 1 sowie deren physiologisch verträglichen Salze als Wachstumsförderer für Tiere.

4. Verwendung von monosilylierten Aminophenylethylamin-Derivaten der allgemeinen Formel (I) gemäß Anspruch 1 in Mengen von 0,01 bis 50 mg pro kg Körpergewicht als Wachstumsförderer für Tiere.

5. Tiernahrung enthaltend monosilylierte Aminophenylethylamin-Derivate der allgemeinen Formel (I) gemäß Anspruch 1.

6. Wachstumsförderndes Mittel für Tiere, enthaltend monosilylierte Aminophenylethylamin-Derivate der allgemeinen Formel (I) gemäß Anspruch 1.

7. Verfahren zur Herstellung von wachstumsfördernder Tiernahrung und Mittel, dadurch gekennzeichnet, daß man monosilylierte Aminophenylethylamin-Derivate der allgemeinen Formel (I) gemäß Anspruch 1 mit Tiernahrung oder Streck- und Verdünnungsmitteln vermischt.

**Claims**

1. Monosilylated aminophenylethylamine derivatives of the general formula (I)

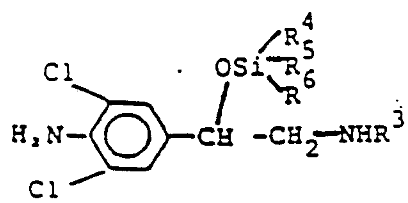

(I)

in which

$R^3$ represents a straight-chain or branched $C_1$-$C_6$-alkyl radical which can be substituted by halogen and

$R^4$, $R^5$ and $R^6$ represent straight-chain or branched alkyl radicals,

and their physiologically tolerated salts.

2. Process for the preparation of monosilylated aminophenylethylamine derivatives of the formula (I)

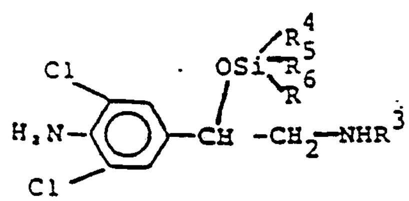

(I)

in which

$R^3$ represents a straight-chain or branched ($C_1$-$C_6$)-alkyl radical which can be substituted by halogen, and

$R^4$, $R^5$ and $R^6$ represent straight-chain or branched alkyl radicals,

characterised in that compounds of the formula II

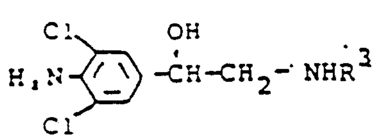

(II)

in which $R^3$ has the meaning given above are reacted, either as the racemate or in one of their enantiomeric forms, with suitable silylating agents of the formula (III)

$$R^4R^5R^6\ Si\text{-}Z \qquad (III)$$

in which

Z denotes halogen, CN, $-O-SO_2-CF_3$, $-O-SiR^4R^5R^6$ or $-O-SO_2-O-SiR^4R^5R^6$, and

$R^4$, $R^5$ and $R^6$ have the meaning given above.

3. Use of monosilylated aminophenylethylamine derivatives of the formula I according to Claim 1 and their physiologically tolerated salts as growth promoters for animals.

4. Use of monosilylated aminophenylethylamine derivatives of the general formula (I) according to Claim 1, in amounts of 0.01 to 50 mg per kg of body weight, as growth promoters for animals.

5. Animal feed containing monosilylated aminophenylethylamine derivatives of the general formula (I) according to Claim 1.

6. Growth-promoting agent for animals, containing monosilylated aminophenylethylamine derivatives of the general formula (I) according to Claim 1.

7. Process for the preparation of growth-promoting animal feed and agent, characterised in that monosilylated aminophenylethylamine derivatives of the general formula (I) according to Claim 1 are mixed with animal feed or extenders and diluents.

**Revendications**

1. Dérivés monosilylés de l'aminophényléthylamine répondant à la formule générale (I)

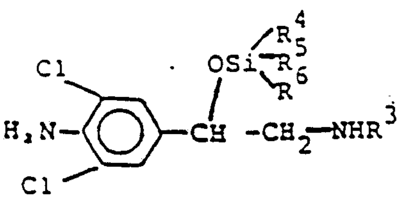

(I)

dans laquelle

$R^3$ représente un radical alkyle en $C_1$-$C_6$ à chaîne droite ou ramifiée, qui peut être substitué par un halogène,

$R^4$, $R^5$, $R^6$ représentent des radicaux alkyles à chaîne droite ou ramifiée,

et leurs sels physiologiquement compatibles.

2. Procédé de production des dérivés monosilylés de l'aminophényléthylamine répondant à la formule (I)

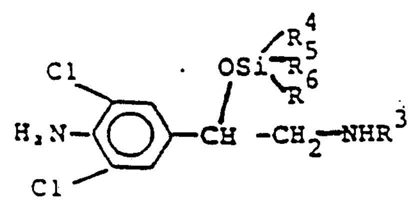

(I)

dans laquelle

$R^3$ représente un radical alkyle en $C_1$-$C_6$ à chaîne droite ou ramifiée qui peut être substitué par un halogène,

$R^4$, $R^5$, $R^6$ représentent des radicaux alkyles à chaîne droite ou ramifiée

caractérisé en ce que l'on fait réagir des composés de la formule (II)

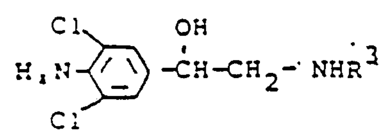

(II)

dans laquelle $R^3$ a la signification mentionnée ci-dessus, soit sous forme de racemate, soit sous forme d'énanthiomères avec des agents de silylation appropriés répondant à la formule (III)

$$R^4R^5R^6\ Si\text{-}Z \qquad (III)$$

dans laquelle

Z représente un halogène, un CN, un $-O-SO_2-CF_3$, un $-O-SiR^4R^5R^6$ ou un $-O-SO_2-O-SiR^4R^5R^6$, où

$R^4$, $R^5$, $R^6$ ont les significations mentionnées ci-dessus.

3. Utilisation de dérivés monosilylés de l'aminophényléthylamine de la formule I, selon la revendication 1 ainsi que de leurs sels physiologiquement compatibles, comme stimulants de la croissance des animaux.

4. Utilisation des dérivés monosilylés de l'aminophényléthylamine de la formule générale (I) selon la revendication 1, en quantités de 0,01 à 50 mg par kg de poids de corps, à titre de stimulants de la croissance des animaux.

5. Aliments pour animaux contenant des dérivés monosilylés de l'aminophényléthylamine de la formule générale (I), selon la revendication 1.

6. Agent de stimulation de la croissance des animaux contenant des dérivés monosilylés de l'aminophényléthylamine de la formule générale (I), selon la revendication 1.

7. Procédé de production d'aliments et de produits stimulant la croissance des animaux, caractérisé en ce que l'on mélange des dérivés monosilylés de l'aminophényléthylamine de la formule générale (I), selon la revendication 1, avec des aliments pour animaux ou des agents de dilution ou de mélange.